# EUROPEAN PATENT SPECIFICATION

(11) **EP 4 037 509 B1**
(45) Date of publication and mention of the grant of the patent: **04.12.2024**
(21) Application number: 20800553.8
(22) Date of filing: 02.10.2020
(51) Int. Cl.: A24F 40/40, A24F 40/42, A61M 11/04, A61M 15/00, A61M 15/06

(54) **AEROSOL GENERATION DEVICE**
AEROSOLERZEUGUNGSVORRICHTUNG
DISPOSITIF DE PRODUCTION D'AÉROSOL

(30) Priority: 02.10.2019 EP 19201113
(43) Date of publication of application: 10.08.2022
(73) Proprietor: JT International SA, 1202 Geneva (CH)
(72) Inventor: MASON, Jon, Maidenhead SL6 7JF (GB); LYELL, Nathan, Woking GU22 7RN (GB); BAKER, Dominic, London SW2 5PT (GB); PLEVNIK, Marko, London NW3 2JR (GB)
(74) Representative: Gill Jennings & Every LLP
(86) International application number: PCT/EP2020/077723
(87) International publication number: WO 2021/064209

(56) References cited:
- WO-A1-2018/224132
- WO-A1-2019/063016
- WO-A1-2019/127896
- GB-A- 2 543 906
- US-A1- 2009 320 838

## Description

### TECHNICAL FIELD

The present disclosure relates to an aerosol generation device and an aerosol generation system comprising an aerosol generation device and consumable.

### TECHNICAL BACKGROUND

Aerosol generation devices such as electronic cigarettes are relatively well known and are becoming increasingly popular with consumers in recent years. A common operating principle for such electronic cigarettes is to heat a consumable without burning it to provide an aerosol (also referred to as a vapour) to a user for inhalation. Examples of such consumables include charges of tobacco material or capsules of liquid containing flavourants and active ingredients such as nicotine.

Aerosol generation devices often comprise a heater, a power source for supplying electricity to the heater and a receptacle, such as a heating chamber, for receiving the consumable in the vicinity of the heater such that the consumable may be heated to produce the vapour for inhalation.

In recent years the popularity of multiple use aerosol generation devices has surpassed that of single use devices which are disposed of after use. Since, the consumable can only provide the required active ingredients or flavourants for a limited duration of heating before these components are depleted, in multiple use devices the consumable must be replaced. This is generally achieved by removing the depleted consumable from the heating chamber and replacing with a new consumable.

There are however several shortcomings with such aerosol generation devices and systems. In particular, the replacement of the consumable is often an awkward process for the user, given the size of the components and the requirement to manoeuvre the aerosol generating device into the appropriate orientation while removing the spent consumable and replacing with a new consumable. This process has to be carried out regularly which exacerbates the problem.

In particular, the consumable may be difficult to remove from the heating chamber because the consumable has been attached to the heating chamber. This attachment may comprise friction between the heating chamber and the consumable, for example if there is a tight fit between the chamber and consumable. Additionally or alternatively, the attachment may comprise a bond formed between the consumable and the chamber when the consumable is heated in the chamber. For example, the consumable may undergo thermal expansion when heated, making it more difficult to remove the consumable after use than it was to insert the consumable for use. As another example, heating the consumable may produce or release a sticky substance such as tar that adheres the consumable to the heating chamber.

Therefore, there is need to provide an aerosol generation device and system which addresses one or more of these shortcomings.

WO 2018/224132 A1 describes a consumable cartridge and an inhaler device. The consumable cartridge comprises a casing defining a cavity, the casing comprising a first region and a second region. An elastic bladder is located within the cavity of said casing and encloses a vaporizing liquid. The first region of the casing comprises a pierceable portion, and the second region comprises a pre-perforated portion. The bladder is disconnected from the casing in the first region, and is configured to rupture or to be cut when it is pierced by a piercing member of the inhaler device such that the ruptured or cut area of the bladder creates an opening which is significantly larger than the cross sectional area of the piercing. When the inhaler device is prepared to be used, the mouthpiece section is separated from the power supply unit and a consumable cartridge is inserted into the cartridge chamber. Once the consumable cartridge is depleted, the inhaler device may be opened and the consumable cartridge can be removed. The consumable cartridge maybe manually removed by pulling it out from the cartridge chamber. In order to facilitate the removal of the consumable cartridge, a grip, such as a protrusion or a flap can be located on a first end region or a second end region of the outer casing of the consumable cartridge. The grip is located on the end region exposed to the user and which is disengaged from the cartridge chamber when the power supply unit is separated from the mouthpiece section. Alternatively, the inhaler device may further comprise a consumable cartridge ejection mechanism. In a simple embodiment, a consumable cartridge ejection mechanism may comprise a resilient member such as a coil spring or a leaf spring. It would also be possible to provide the inhaler device with a mechanical cartridge ejection mechanism, which may be activated by a lever.

### SUMMARY OF THE DISCLOSURE

According to a first aspect of the present disclosure, an aerosol generation device comprises a chamber, a housing and a cover. The chamber is adapted to receive a consumable. The housing contains the chamber and comprises an opening through which the consumable can be removed from the chamber. The cover is configured to move between an open position where the opening of the housing is exposed and a closed position where the opening of the housing is closed. The aerosol generation device is adapted to generate an inhalable vapor from the consumable. The cover comprises one or more dislodging elements adapted to dislodge the consumable such that, when the cover moves from the closed position to the open position: an attachment is weakened between the chamber and the consumable, and the consumable is partially ejected from the chamber without becoming attached to the cover.

Optionally, the attachment comprises: friction between the consumable and the chamber; and/or a bond formed between the consumable and the chamber when the consumable is heated in the chamber.

Optionally, the open position is configured such that a new consumable can be inserted through the opening, and the closed position is configured such that a user may operate the aerosol generation device to generate and inhale the inhalable vapor from the consumable.

Optionally, the one or more dislodging elements comprise two or more dislodging elements.

Optionally, the dislodging elements are adapted to engage with a flange part of the consumable.

Optionally, the dislodging elements are snap-fit connectors.

Optionally, the one or more dislodging elements each comprise an engagement member configured to move substantially perpendicular to a direction of motion of the cover so as to form a snap-fit connection.

Optionally, the engagement member is a resilient member.

Optionally, the cover comprises a resilient seal arranged such that, when the cover is in the closed position, the resilient seal contacts the flange part of the consumable.

Optionally, the aerosol generation device comprises a sensor arranged to detect when the cover is pressed against the housing, and the resilient seal is arranged to bias the cover away from the housing.

Optionally, the aerosol generation device further comprises control circuitry configured to control the aerosol generation device to start generation of the inhalable vapor when the sensor detects that the cover is pressed against the housing.

Optionally, the cover is attached to the housing in the open position.

Optionally, the cover is attached to the housing by a hinge.

Optionally, the hinge comprises a detent axle configured such that the cover is stable at each of the open position and the closed position.

Optionally, the hinge comprises a torsion spring, and a magnet is comprised in the cover or the housing, wherein the torsion spring is configured to bias the cover towards the open position, and the magnet is configured to hold the cover in the closed position.

Optionally, the one or more dislodging elements are each arranged to partially oppose the hinge.

Optionally, a first dislodging element of the one or more dislodging elements is arranged such that, when the first dislodging element dislodges the consumable, the consumable is between the first dislodging element and the hinge.

Optionally, the aerosol generation device is elongate along a longitudinal direction, and the hinge is oriented perpendicular to the longitudinal direction.

Optionally, the cover comprises a mouthpiece and a flow channel through which the inhalable vapor can flow.

Optionally, the housing comprises an abutting surface against which the cover can abut, the surface comprising the opening, and the chamber is recessed within the housing relative to the abutting surface.

Optionally, the dislodging element is arranged to extend through the opening when the cover is in the closed position.

### BRIEF DESCRIPTION OF THE DRAWINGS

Figures 1A to 1C are schematic illustrations of an aerosol generation device;
Figures 2A to 2E are schematic illustrations of a dislodging process in an aerosol generation device;
Figures 3A to 3C are schematic illustrations of dislodging elements in an aerosol generation device;
Figures 4A to 4C are schematic illustrations of an aerosol generation device having a hinged cover;
Figures 5A and 5B are schematic illustrations of an aerosol generation device having a hinged cover including a detent axle;
Figures 6A and 6B are schematic illustrations of an aerosol generation device having a hinged cover including a torsion spring;
Figures 7A and 7B are schematic illustrations of an aerosol generation device having an indicator;
Figures 8A, 8B and 8C are schematic illustrations of an aerosol generation device having a control system;
Figure 9 is an exploded view of an aerosol generation device.

### DETAILED DESCRIPTION

Embodiments of the present disclosure will now be described by way of example only and with reference to the accompanying drawings.

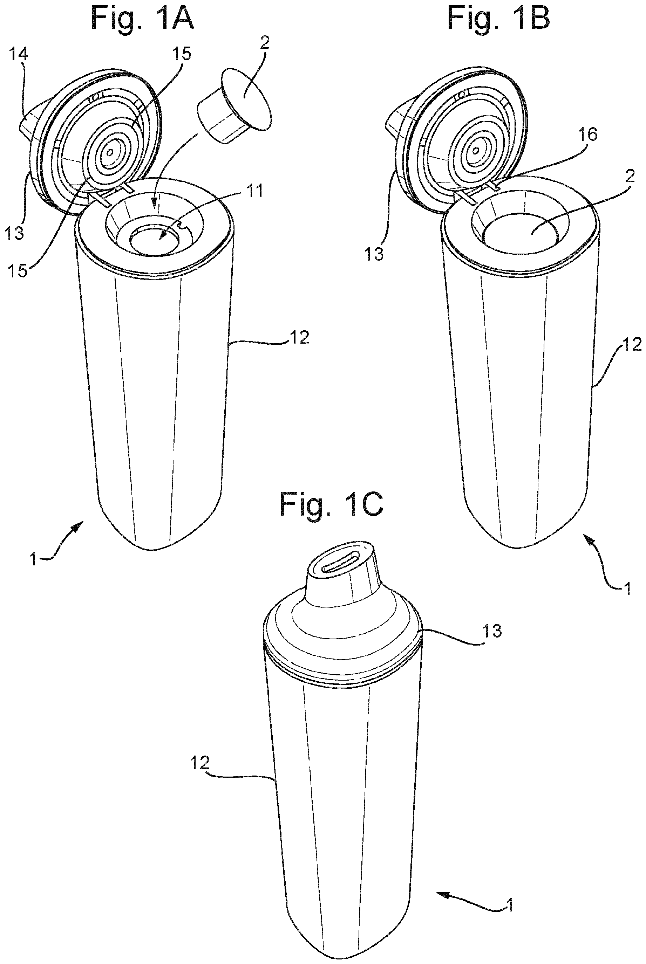

Figures 1A to 1C schematically illustrate an aerosol generation device 1 according to the present invention. The aerosol generation device 1 comprises a chamber 11 adapted to receive a consumable 2, and further comprises a housing 12 and a cover 13. Figs. 1A to 1C also sequentially illustrate a method of inserting the consumable into the aerosol generation device in preparation for generating an inhalable vapor.

As shown in Figs. 1A and 1B, the chamber 11 is contained in the housing 12, which has an opening through which the consumable 2 can be added to or removed from the chamber 11. The housing may be an elongate housing adapted for a user to hold the aerosol generation device.

The chamber 11 is a heating chamber for containing the consumable while it is heated by a heater (not shown) that is also within the housing 12. In some embodiments, the heater may additionally or instead be included in the cover 13.

The cover 13 is configured to close the opening of the housing 12 when it is in a closed position as shown in Fig. 1C, and is configured to move between the closed position and an open position where the opening of the housing is exposed as shown in Figs. 1A and 1B. In the open position, the cover 13 is out of the way so that a consumable 2 can be inserted in or removed from the chamber 11 through the opening of the housing 12.

The cover 13 comprises one or more dislodging elements 15 adapted to dislodge the consumable 2 when the cover moves from the closed position to the open position. In this specification, "dislodge" means that the dislodging elements 15 weaken an attachment between the chamber 11 and the consumable 2. Due to this weakening of the attachment, the consumable is partially ejected from the chamber. This means that, as the cover opens, the consumable may either be loosened while remaining in the chamber 11 or may pop out from the aerosol generation device 1 due to the force exerted by the dislodging elements 15. However, the dislodging elements 15 are not configured to hold the consumable 2 in the open position, and the consumable 2 does not become attached to the cover 13. The dislodging elements 15 may be configured to hold the consumable 2 at or near the closed position and release the consumable 2 as the cover 13 moves to the open position. Alternatively, the dislodging elements 15 may be configured not to hold the consumable 2 at all. In this case, the dislodging elements 15 are further configured to brush past the consumable 2 as the cover 13 moves from the closed position to the open position and thereby perform the dislodging.

As an example, a user may suspend the aerosol generation device over a waste collection apparatus (e.g. a bin) with the closed cover directed towards the waste collection apparatus such that, when the cover moves from the closed position to the open position (i.e. the cover opens), the consumable 2 is dislodged by the dislodging elements 15 and falls into the waste collection apparatus. Alternatively, the user may hold the aerosol generation device with the closed cover directed upwards such that, when the cover moves from the closed position to the open position, the consumable 2 is dislodged by the dislodging elements 15 and partially ejected from the chamber 11 but remains partially in the chamber 11 or rests on the housing 12 such that the user may easily remove the consumable 2.

The aerosol generation device 1 is adapted to generate an inhalable vapor from the consumable 2, and the aerosol generation device 1 and the consumable 2 can be regarded together as constituting an aerosol generation system or as a kit for aerosol generation. More specifically, when the consumable 2 is in the chamber 11 and is heated by the heater, a vapor is generated. The vapor passes to a mouthpiece 14 of the aerosol generation device 1 where it can be inhaled by a user of the aerosol generation device 1. In this embodiment, the mouthpiece 14 forms part of the cover 13, but in other embodiments the mouthpiece 14 may form part of the housing 12, or may be replaced with an alternative means for releasing the inhalable vapor in uses of the invention where a mouthpiece is not required. The mouthpiece 14 may be made from a soft or flexible material in order to be comfortable for a user.

The cover 13 may additionally be adapted such that, in the closed position, the user may operate the aerosol generation device 1 to generate and inhale the inhalable vapor from the consumable 2. On the other hand, the aerosol generation device 1 may be adapted to disable generation of the vapor when the cover 13 is in the open position.

In this embodiment, the cover 13 is attached to the housing 12, even when the cover 13 is in the open position. More specifically, the cover 13 is attached to the housing 12 by a hinge 16. Additionally, in the illustrated embodiment, the hinge 16 is oriented perpendicular to a longitudinal direction of the elongate aerosol generation device (i.e. a direction through the mouthpiece 14 when the cover 13 is in a closed position and through a base of the housing 12). In alternatives, the cover 13 may be detachable such that the open position includes any position where the opening of the housing 12 is exposed. Furthermore, the cover 13 may be permanently attached to the housing 12 without being constrained to one specific open position, for example if the cover 13 is attached to the housing 12 via a flexible member or a string. More generally the only required constraint on the movement of the cover 13 is that, when the cover 13 moves from the closed position, the dislodging elements 15 dislodge the consumable 2.

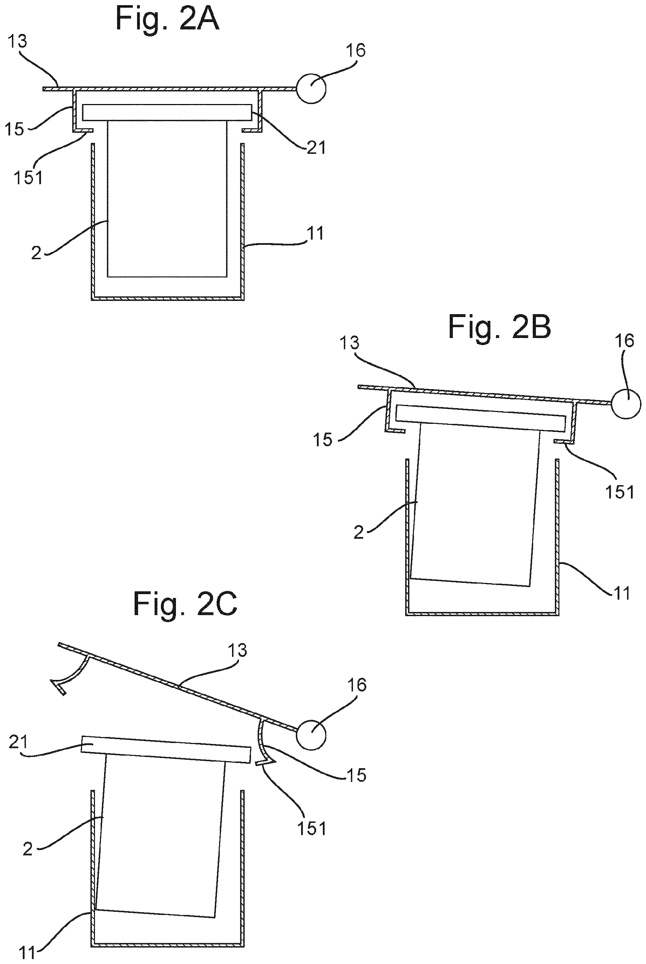

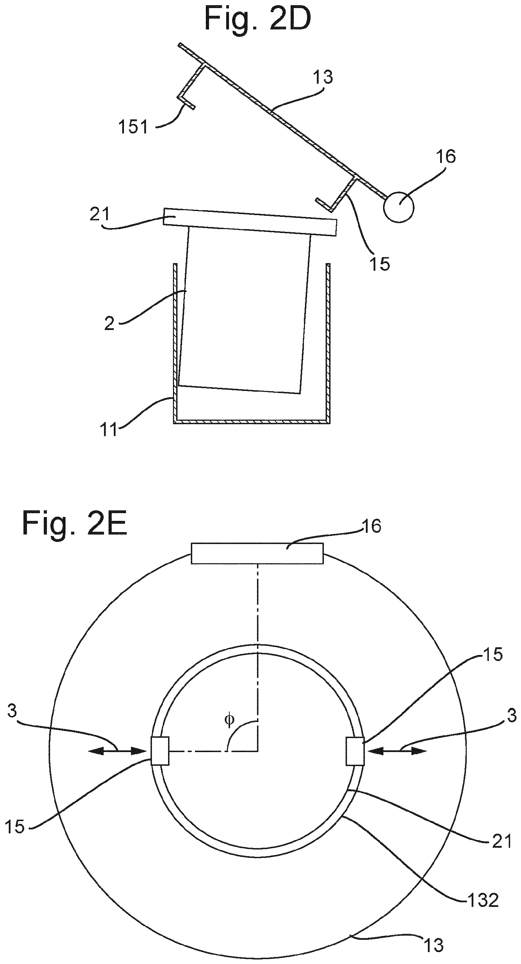

Figures 2A to 2E schematically show a consumable 2 and elements of an aerosol generation device in order to illustrate the principle of operation of the one or more dislodging elements 15.

More specifically, Fig. 2A illustrates a cross-section of the aerosol generation system in which a cover 13 is in a closed position over a chamber 11 containing a consumable 2. Two dislodging elements 15 are adapted to engage with a flange part 21 of the consumable 2.

Although two dislodging elements 15 are shown in this example, the dislodging effect of the invention may be achieved with only one dislodging element 15 or with more than two dislodging elements 15. However, using at least two dislodging elements has the advantage of improving control of how the consumable 2 moves when dislodged from the chamber 11, while minimizing the number of dislodging elements to be no more than two has the advantage of simplifying manufacturing of the aerosol generation device 1.

As shown successively in Figures 2B to 2D, when the cover 13 moves from the closed position to the open position, in this case pivoting around hinge 16, the dislodging elements 15 engage with the flange 21 and thereby exert a force on the consumable 2 to dislodge the consumable from the chamber 11. However, this force on the consumable 2 also biases the dislodging elements toward disengaging with the flange 21 and, by the time the cover 13 arrives at the open position, the dislodging elements 15 are disengaged from the flange 21 and the consumable 2 does not become attached to the cover 13 in the open position. Thus, it will be understood that the dislodging elements 15 have a flicking effect which lightly grips the flange 21 when the cover 13 is in the closed position and, when the cover 13 moves from the closed position to the open position, the dislodging elements have a slight dislodging effect which ensures that the consumable is not stuck in place in the chamber 11.

The dislodging elements 15 may be snap-fit connectors. The dislodging elements may each comprise an engagement member 151 configured to move substantially perpendicular to a direction of motion of the cover. The ability of the engagement members to undergo this substantially perpendicular motion allows the dislodging elements 15 to engage with the flange 21 and form a snap-fit connection. This ability also causes the dislodging elements 15 to disengage from the flange 21 as the cover 13 moves to the open position. Here "substantially perpendicular" means that that the engagement member moves sideways as shown in Fig. 2C. In particular, snap-fit connectors are often resilient members that work by bending before snapping into place as shown in Fig. 2C, and such bending motion includes a component of motion which is antiparallel to the direction of motion when forming the snap-fit connection. Accordingly, in such cases, the motion is not truly perpendicular but is nevertheless substantially perpendicular.

In other embodiments, the above described perpendicular motion for engaging and disengaging could instead be achieved by mechanical or electronic control systems which control the entire engagement member to move at appropriate positions for engaging and disengaging, rather than relying on natural bending of a resilient member. For example, a motion of the engagement member could be mechanically linked to the angle of the hinge 16 between the housing 12 and the cover 13.

Figure 2E schematically illustrates a plan view of the cover 13 including dislodging elements 15 engaged with a flange 21 of a consumable. This represents an inside view of the cover in the closed position. Fig. 2E illustrates an embodiment where both the cover 13 and the consumable 2 have a circular cross-section. However, this need not be the case, and both of the aerosol generation device and the consumable may, for example, instead have a polygonal cross-section.

Figure 2E illustrates that the dislodging elements 15 may be arranged around a protruding part 132 of an inner surface of the cover 13, and illustrates a direction of motion 3 of the dislodging elements 15 in the plan view. This direction 3 is towards and away from a centre of the cover 13 (i.e. a radial motion in the case of a circular cross-section). An angle between a dislodging element 15 and the hinge 16, relative to the centre of the cover 13, is labelled as phi φ. It should be noted that phi φ for the dislodging elements 15 in Figs. 2A to 2D is 0 degrees and 180 degrees, whereas phi φ for both of the dislodging elements 15 in Fig. 2E is 90 degrees.

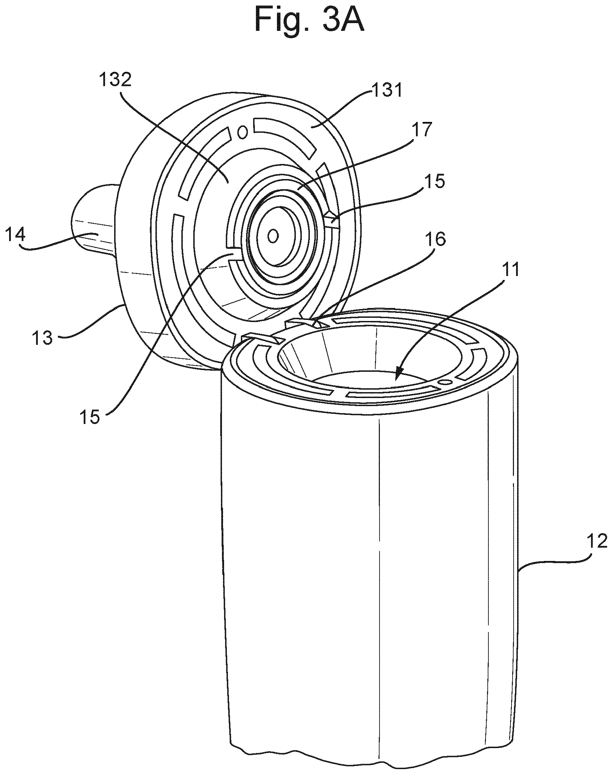

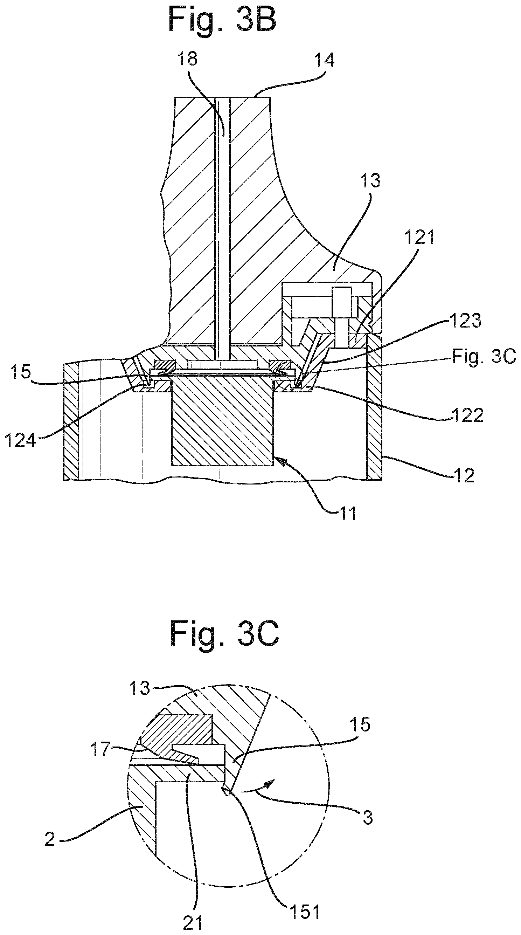

Figures 3A to 3C schematically illustrate additional details of the aerosol generation device 1 of this embodiment. It should be noted that Fig. 3B is a cut-away view where the hinge is omitted.

In particular, as shown in Fig. 3A, the cover 13 comprises a resilient seal 17. This seal is arranged such that, when the cover is in the closed position shown in Figs. 3B and 3C, the seal contacts the flange part 21 of the consumable 2. For example, the resilient seal 17 may be an elastomeric lip seal.

Additionally, as shown in Fig. 3B, the cover 13 comprises a flow channel 18 through which inhalable vapor can flow from the consumable 2 to the mouthpiece 14. When the consumable 2 is designed to generate inhalable vapor at a top surface surrounded by the flange 21, the resilient seal 17 ensures that the inhalable vapor flows through the flow channel 18 to the mouthpiece 14, and does not escape through any gap between the cover 13 and the housing 12.

In this embodiment, the seal 17 is mounted on the protruding part 132 of the inner surface of the cover 13, the protruding part being surrounded by a flat part 131. The protruding part also comprises the dislodging elements 15. Furthermore, in this embodiment, the dislodging elements 15 form smooth extensions of an outer surface of the protruding part 132, making the dislodging elements 15 more robust and less vulnerable to snapping off.

Correspondingly, as shown in Fig. 3B, in this embodiment an inner surface of the housing 12 comprises an abutting surface 121 against which the cover 13 can abut. The abutting surface 121 comprises the opening through which the consumable can be added to or removed from the chamber 11, where the chamber 11 is recessed within the housing 12 relative to the abutting surface 121. For example, the inner surface of the housing 12 may further comprise a consumable support surface 122 arranged adjacent to the chamber 11 to support the consumable 2 in the chamber, and a connecting wall 123 between the abutting surface 121 and the consumable support surface 122.

When the cover 13 is in the closed position, the flat part 131 of the inner surface of the cover aligns with the abutting surface 121, and the dislodging elements 15 (and optionally the protruding part of the inner surface of the cover 13) extend through the opening.

The consumable support surface 122 may be adapted with an additional recess 124 as shown in Fig. 3B to enable an engagement means 151 of the dislodging element 15 to extend past and engage with the consumable 2 while it is supported by the consumable support surface 122.

As further shown in Fig. 3C, the engagement members 151 of this embodiment take the form of protrusions arranged to catch the edges of the flange 21 of consumable 2. In this figure, arrow 3 (previously shown in the plan view of Fig. 2E) illustrates the motion of the dislodging element 15 in a vertical cross-section view. More specifically, the dislodging element bends radially outward and bends upwards towards the cover 13 to allow the engagement member 151 to disengage from the flange 21 when the consumable 2 has been dislodged.

Referring again to Fig. 3A, it can also be seen that, in this embodiment, the dislodging elements 15 are each arranged to partially oppose the hinge 16. In other words, when the dislodging elements dislodge the consumable, the consumable is between each dislodging element and the hinge. It should be understood that, here, "between" has a broad meaning that includes the case where the consumable 2 is between any part of the dislodging element 15 and any part of the hinge 16, for example as shown previously in Fig. 2E. More specifically, each of the dislodging elements 15 and the hinge 16 are arranged around a centre of the cover 13, where the angle phi φ around the centre of the cover 13 between the hinge 16 and each of the dislodging elements 15 is at least 90 degrees. For example, the angle phi φ for a dislodging element 15 may be 90, 100, 120, 130, or 180 degrees. By arranging the dislodging elements 15 in this way to be on an opposing side of the consumable 2 from the hinge 16, the force applied by each dislodging element 15 on the flange 21 and vice versa is reduced for a given torque applied to open the cover 13 around the hinge 16. This means that the energy required to bend the engagement member 151 to release the consumable 2 is applied over a longer distance and weaker materials can be used for the dislodging elements 15 without breaking when dislodging the consumable 2. Additionally, by arranging the dislodging elements at least 90 degrees from the hinge 16, the dislodging elements are not arranged on one side of the consumable, and the consumable is pushed towards a wall of the chamber 11 and towards the consumable support surface 122 when the cover 13 rotates around the hinge 16. This increases the friction between the consumable 2 and the wall of the chamber 11 or the consumable support surface 122 until the dislodging elements 15 experience enough resistance to bend and disengage from the flange 21. It should be noted that this need not be the case for all of the dislodging elements 15 and an advantage is obtained if even one of the dislodging elements is located at an angle phi φ of at least 90 degrees. For example, as shown in Figs. 2A to 2D, the angle phi φ for the left dislodging element in each figure is greater than 90 degrees (specifically 180 degrees) and the angle phi φ for the right dislodging element in each figure is less than 90 degrees (specifically 0 degrees).

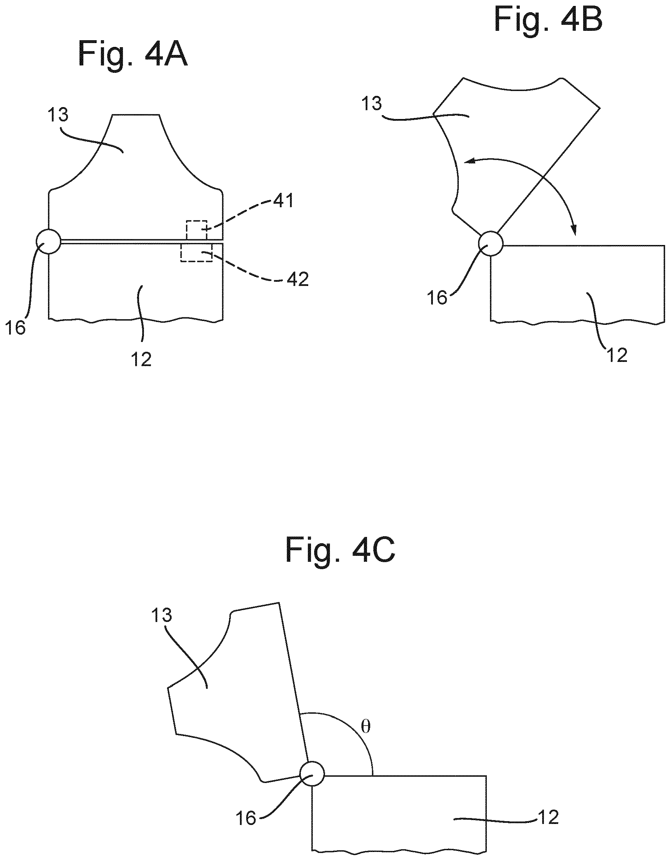

Referring to Figs. 4A to 4C, additional details of the hinged motion of the aerosol generation device of the present embodiment are illustrated. Fig. 4A illustrates the cover 13 in the closed position relative to the housing 12, Fig. 4B illustrates the cover 13 moving around the hinge 16 between the closed position and the open position, and Fig. 4C illustrates the cover 13 in the open position relative to the housing 12. As shown in Fig. 4C, in the open position, the angle theta θ between the cover 13 and the housing 12 may be greater than 90 degrees, which has the advantage that it is easy to access the consumable 2 in the chamber 11.

In this embodiment, the hinge 16 is adapted to be bistable with the cover 13 in the closed position of Fig. 4A and the open position of Fig. 4C, while being unstable in an intermediate position such as shown in Fig. 4B. This means that the hinge 16 will snap to either the open position or the closed position, and is biased against remaining in an intermediate position between the open position and the closed position.

The cover 13 and housing 12 may respectively comprise a Hall effect magnet 41 and a Hall effect sensor 42 as shown in Fig. 4A. The Hall effect sensor detects the presence of the Hall effect magnet and thus detects when the cover 13 is in the closed position.

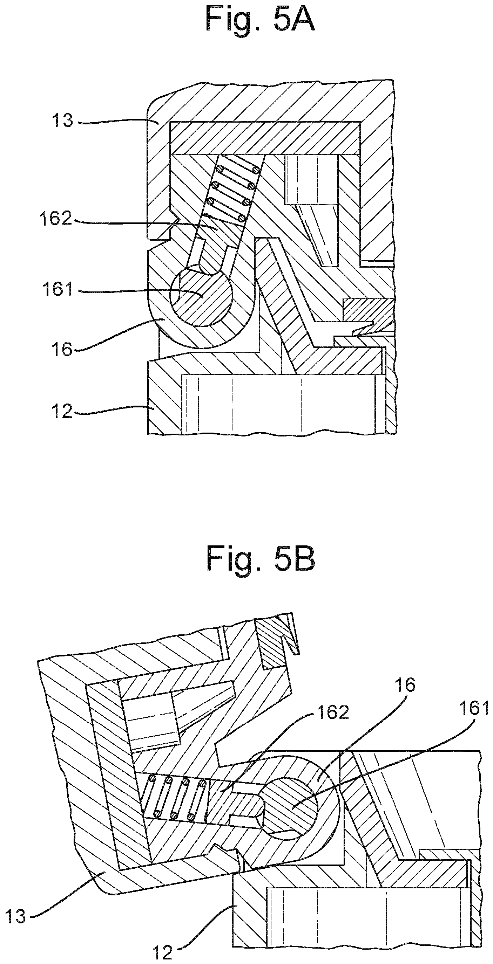

Two embodiments of the bistable hinge 16 are illustrated using Figs. 5A to 6B. However, other bistable hinges may be used. Furthermore, in embodiments where a hinge 16 is included, it is not necessary for the hinge to be bistable, and the hinge may be free around the open position. It is however advantageous for the hinge to be stable at least in the closed position so that the aerosol generation device is less likely to accidentally open.

Firstly, in Figs. 5A and 5B, a bistable hinge 16 comprises a detent axle 161 together with a sprung plunger 162. The spring of the sprung plunger 162 biases the plunger towards the detent axle 161, and one of the detent axle 161 and the sprung plunger 162 is fixed as the cover 13 moves between the closed position and the open position, meaning that the plunger 162 moves around the detent axle 161. Recesses in the detent axle 161 provide stable positions at which the plunger 162 can extend further towards the detent axle 161, and moving between these stable positions requires compression of the spring of the sprung plunger 162. Thus, by configuring the detent axle with a respective indent at each of the closed position and the open position, a bistable hinge is achieved wherein the cover 13 is stable at each of the open position and the closed position. A further advantage of the detent axle embodiment is that it may be entirely concealed within a hinge assembly. It should be noted that the sprung plunger 162 is not essential for embodiments which use a detent axle 161 and could be replaced with any biasing means for biasing towards the indents of the detent shaft. Furthermore, the embodiment could be inverted such that the detent shaft has indents on an internal surface which rotates around an axle comprising the biasing means.

This type of bistable hinge requires that the plunger 162 must pass a peak between the two indents in the detent axle 161 in order to switch between the two stable positions. Therefore, there is a minimum angle by which the cover 13 must be opened before it will stably settle on the open position when starting from the closed position. This minimum angle is set according to the shape of the detent axle but is half of the total range of motion in the case of a symmetrical detent axle.

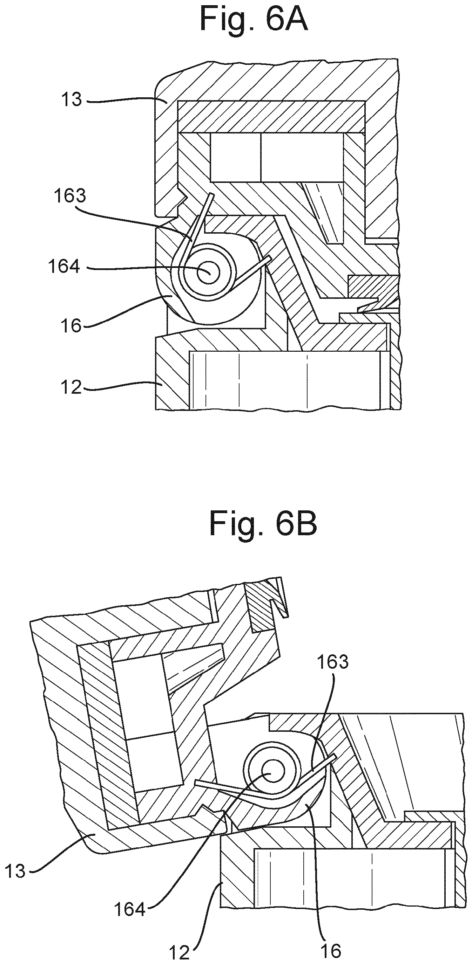

Secondly, in Figs. 6A and 6B, a bistable hinge 16 comprises a torsion spring 163 and an axle pin 164. In this case, the torsion spring is configured to bias the cover 13 towards the open position, and the stable open position is fixed by a limit of the range of motion of the cover 13 around the hinge 16 due to contact with the housing 12. However, a torsion spring 163 configured with such a bias cannot also provide a stable closed position. Therefore, this embodiment also comprises one or more magnets in the cover 13 and/or the housing 12 arranged such that, when the cover 13 is in the closed position, the cover 13 is attracted to the housing 12 with a force that balances the bias provided by the torsion spring 163, and is held in the closed position. The strength of the magnet(s) can be set such that, when the cover 13 moves even a small distance from the closed position, the force of the torsion spring 163 exceeds the force of the magnet(s), and the cover 13 will spring to the open position. Thus, the bistable hinge of this embodiment could instead be described as "semi bistable".

Comparing the above two bistable hinge embodiments with respect to the action of the one or more dislodging elements 15, in the case of the detent axle of Figs. 5A and 5B, a user must provide the force to both overcome the sprung plunger 162 and to drive the dislodging action of the dislodging elements, because half of the total range of motion of the cover 13 would in most cases be further than the consumable 2 will move when dislodged. On the other hand, in the case of the spring hinge of Figs. 6A and 6B, the user need only overcome the force of the magnets holding the cover 13 in the closed position. After this force has been overcome, the torsion spring 163 provides the force to drive the dislodging action of the dislodging elements 15 and move the cover 13 to the open position. Therefore, by providing a spring hinge as in Figs. 6A and 6B with magnets chosen to be as weak as possible while being capable of holding the cover 13 in the closed position, an aerosol generation device can be provided where the cover 13 is easy to open and where the consumable 2 is easily removed after the cover 13 has been opened (or at the same time as the cover 13 is opened if, for example, the aerosol generation device is held with the cover 13 pointing downward in the closed position).

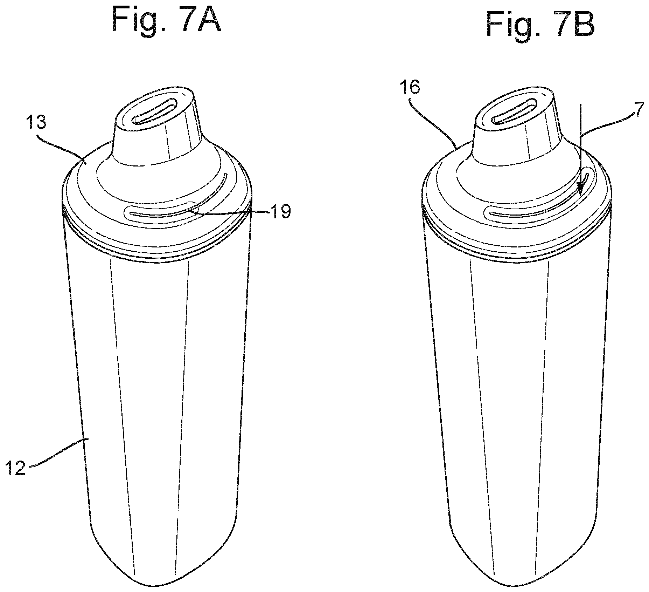

Figures 7A and 7B schematically illustrate an indicator 19 of an embodiment. The indicator may, for example, indicate an amount of energy remaining in a power supply of the aerosol generation device, or may indicate the presence or absence of a consumable 2 in the aerosol generation device.

Furthermore, the indicator 19 may indicate a position for a user to operate the aerosol generation device. More specifically, if, as illustrated in Fig. 7B, the indicator 19 is located on an opposing side of the cover 13 from a hinge 16 between the cover 13 and the housing 12, then the indicator 19 may indicate a position at which the user can press the cover 13 against the housing 12 in order to start heating the consumable 2 in the chamber 11. In this same position, the indicator 19 can indicate where the user should push in order to rotate the cover 13 around the hinge 16 in order to move the cover 13 from the closed position to the open position.

Thus the indicator 19 may simultaneously provide many functions of indicating a status of the aerosol generation device, indicating how to control aerosol generation by the device and indicating how to open the aerosol generation device.

Advantageously, the aerosol generation device 1 may be configured such that it can be held in one hand along its elongate housing 12 and the cover 13 may be operated with a thumb where, if the thumb presses against the mouthpiece 14, the cover 13 moves from the closed position to the open position and, if the thumb presses down on the indicator 19, the aerosol generation device starts heating the consumable 2.

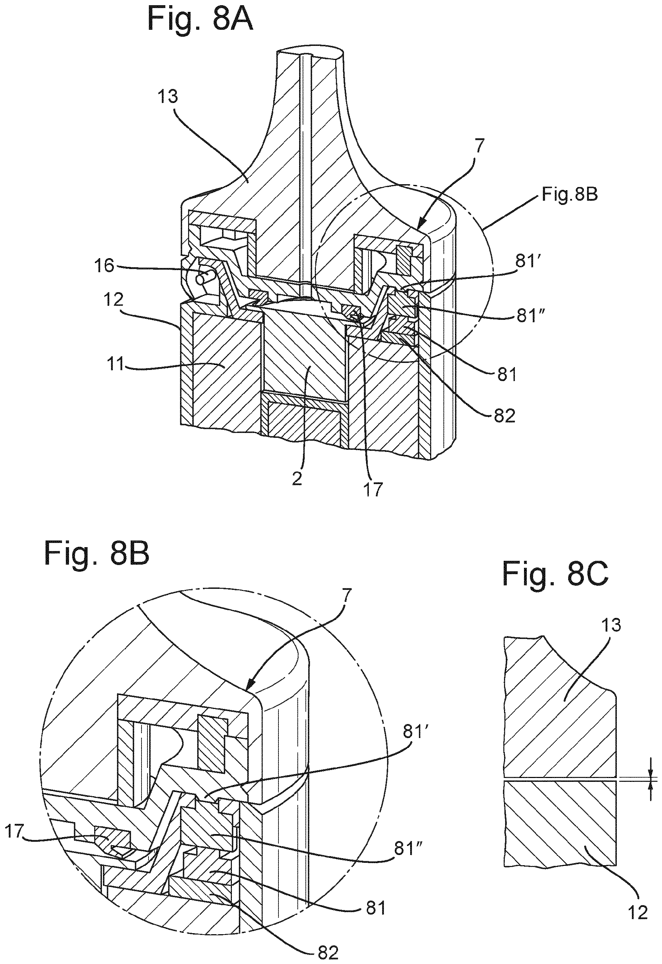

Figures 8A to 8C illustrate further details of a mechanism for controlling an aerosol generation device. Fig. 8A shows a cross-section of the aerosol generation device, Fig. 8B shows magnified portion of the cross-section including the mechanism for controlling the aerosol generation device, and Fig. 8C illustrates the relative positioning of the cover 13 and the housing 12.

As illustrated in Fig. 8A, in this embodiment, while the chamber 11 surrounds a consumable 2 that has been inserted in the device, the chamber 11 does not fill an internal space of the housing 12 and there may be space within the housing 12, for example below the consumable 2 as shown in Fig. 8A, in which other components such as control circuitry and an electrical power source are accommodated.

Fig. 8A also illustrates that there is some clearance between the consumable support surface 122 and the inner surface of the cover 13 which is provided by the consumable 2 and the seal 17. Similarly, Fig. 8C illustrates that there is some clearance between the abutting surface 121 and the flat part 131 of the inner surface of the cover 13.

Due to these clearances, it is possible for the cover 13 to pivot on the hinge 16 towards the housing 12, when pressed as shown using arrow 7, even when starting from the closed position. Thus, in such embodiments, it is useful to include a sensor 81 arranged to detect when the cover 13 is pressed against the housing 12. The sensor 81 may for example be a tactile switch. In this embodiment, the sensor 81 is arranged on a PCB 82, and a corresponding notch 81' in the cover 13 and extension portion 81" transfer a pressing force to the sensor 81. This pressing is only detected when the cover 13 is actively pressed against the housing 12 by the user, because the seal 17 is arranged to bias the cover 13 away from the housing 12. Accordingly, the combination of the seal 17 and the sensor 81 provides a button control for the aerosol generation device. It should be noted that, in other embodiments, the seal 17 need not be resilient, and the biasing of the cover 13 away from the housing 12 could instead be provided, for example, by a torsion spring 163 in the hinge 16.

Such a button control may be combined with control circuitry configured to control the aerosol generation device to start generation of the inhalable vapor when the sensor 81 detects that the cover 13 is pressed against the housing 12. For example, control circuitry may be provided in the PCB 82 to control a heater for heating the consumable 2 in the chamber 11.

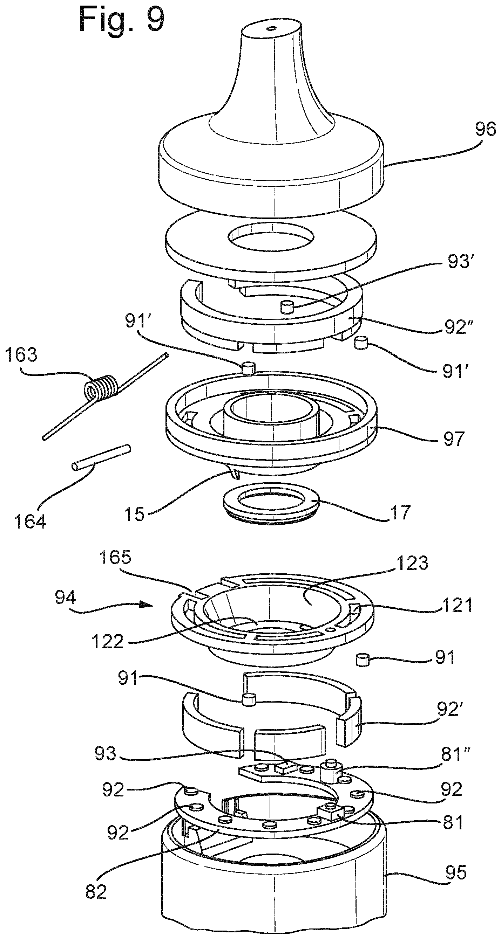

Figure 9 is a schematic exploded view of an aerosol generation device showing additional optional features beyond those described above. In the embodiment shown in Fig. 9, the hinge 16 comprises a torsion spring and axle pin as in Fig. 6, but the features of Fig. 9 may be combined with other embodiments such as embodiments using a detent axle in the hinge.

Fig. 9 shows closure magnet pairs 91, 91' provided in the housing 12 and the cover 13 for holding the cover 13 in the closed position. Two magnet pairs are shown in this embodiment, but any number of pairs may be used. It is advantageous to provide magnet pairs which are arranged symmetrically relative to the hinge 16 so that each magnet pair contributes equally to holding the cover 13 in the closed position.

Fig. 9 also shows LEDs 92 which provide the indicator 19 in this embodiment. The LEDs 92 are mounted on the PCB 82, and light from the LEDs is guided through one or more body light guides 92' in the housing 12 and one or more top light guides 92" in the cover 13, so that light from the LEDs is visible to a user as the indicator 19 shown in Figs. 7A and 7B, when the cover 13 is in the closed position. Referring back to Fig. 3A, it can be seen that, when the aerosol generation device is assembled, the light guides 92' and 92" are flush with the inner surfaces of the cover 13 and the housing 12.

Fig. 9 also shows a Hall effect sensor 93 mounted on PCB 82, and a Hall effect magnet 93' arranged in the cover 13, which are similar to the Hall effect sensor and Hall effect magnet shown in Fig. 4A. The Hall effect sensor 93 may be used by control circuitry to detect when the cover 13 is in the closed position. The control circuitry may thereby detect when the indicator 19 can be generated using the LEDs and light guides 92, 92', 92" or when generation of the inhalable vapor can be started.

As further shown in Fig. 9, the housing 12 may be provided in two parts with a separate inner surface 94 comprising the abutting surface 121, the consumable support surface 122 and the connecting wall 123. This may connect into an outer housing 95 using, for example, a snap fit or press fit connection when the device is assembled during manufacturing. The separate inner surface 94 or the outer housing 95 may further comprise a hinge engaging part 165 adapted to connect with the hinge 16.

Similarly, the cover 13 may be provided in two parts with a separate outer part 96 including the mouthpiece 14 and the flow channel 18, and a separate inner surface 97 including the dislodging element(s) 15 and to which the seal 17 may be attached.

By providing separate parts for the housing and/or the cover, the individual parts of the housing/cover may be manufactured separately and then assembled around the inner components of the housing/cover (i.e. assembled around the chamber 11, the PCB 82, the light guides, magnets etc.).

As the skilled person will recognise, many features in the above-described embodiments have benefits which are independent from other features. Thus the skilled person will recognise that features with individual benefits may be individually included in or omitted from embodiments, so long as they fall within the scope of the invention as defined by the attached claims.

## Claims

1. An aerosol generation device (1) comprising:
a chamber (11) adapted to receive a consumable (2);
a housing (12) containing the chamber (11) and comprising an opening through which the consumable (2) can be removed from the chamber (11); and
a cover (13) configured to move between an open position where the opening of the housing (12) is exposed and a closed position where the opening of the housing (12) is closed;
wherein the aerosol generation device (1) is adapted to generate an inhalable vapor from the consumable (2)
**characterized in that** the cover (13) comprises one or more dislodging elements (15) adapted to dislodge the consumable (2) such that, when the cover (13) moves from the closed position to the open position:
an attachment is weakened between the chamber (11) and the consumable (2), and
the consumable (2) is partially ejected from the chamber (11) without becoming attached to the cover (13).

2. An aerosol generation device (1) according to claim 1, wherein the attachment comprises:
friction between the consumable (2) and the chamber (11); chamber, and/or
a bond formed between the consumable (2) and the chamber (11) when the consumable (2) is heated in the chamber (11).

3. An aerosol generation device (1) according to any preceding claim, wherein the open position is configured such that a new consumable (2) can be inserted through the opening, and the closed position is configured such that a user may operate the aerosol generation device (1) to generate and inhale the inhalable vapor from the consumable (2).

4. An aerosol generation device (1) according to any preceding claim, wherein the dislodging elements (15) are adapted to engage with a flange part (21) of the consumable (2).

5. An aerosol generation device (1) according to claim 4, wherein the dislodging elements (15) are snap-fit connectors.

6. An aerosol generation device (1) according to claim 5, wherein the one or more dislodging elements (15) each comprise an engagement member (151) configured to move substantially perpendicular to a direction of motion of the cover (13) so as to form a snap-fit connection.

7. An aerosol generation device (1) according to claim 6, wherein the engagement member (151) is a resilient member.

8. An aerosol generation device (1) according to any of claims 4 to 7, wherein the cover (13) comprises a resilient seal (17) arranged such that, when the cover (13) is in the closed position, the resilient seal (17) contacts the flange part (21) of the consumable (2).

9. An aerosol generation device (1) according to claim 8, wherein the aerosol generation device (1) comprises a sensor (42) arranged to detect when the cover (13) is pressed against the housing (12), and the resilient seal (17) is arranged to bias the cover (13) away from the housing (12).

10. An aerosol generation device (1) according to any preceding claim, wherein the cover (13) is attached to the housing (12) in the open position.

11. An aerosol generation device (1) according to claim 10, wherein the cover (13) is attached to the housing (12) by a hinge (16).

12. An aerosol generation device (1) according to claim 11, wherein the hinge (16) comprises a detent axle (161) configured such that the cover (13) is stable at each of the open position and the closed position.

13. An aerosol generation device (1) according to claim 11, wherein the hinge (16) comprises a torsion spring (163), and a magnet (41) is comprised in the cover (13) or the housing (12), wherein the torsion spring (163) is configured to bias the cover (13) towards the open position, and the magnet (41) is configured to hold the cover (13) in the closed position.

14. An aerosol generation device (1) according to any of claims 11 to 13 wherein a first dislodging element (15) of the one or more dislodging elements is arranged such that, when the first dislodging element (15) dislodges the consumable (2), the consumable (2) is between the first dislodging element (15) and the hinge (16).

15. An aerosol generation device (1) according to any of claims 11 to 15 wherein the aerosol generation device (1) is elongate along a longitudinal direction and the hinge (16) is oriented perpendicular to the longitudinal direction.

16. An aerosol generation device (1) according to any preceding claim, wherein the housing (12) comprises an abutting surface (121) against which the cover (13) can abut, the surface comprising the opening, and the chamber (11) is recessed within the housing (12) relative to the abutting surface (121).

17. An aerosol generation device (1) according to claim 16, wherein the dislodging element (15) is arranged to extend through the opening when the cover (13) is in the closed position.

## Patentansprüche

1. Aerosolerzeugungsvorrichtung (1) umfassend:
Eine Kammer (11), die zur Aufnahme eines Verbrauchsmaterials (2) geeignet ist;
ein Gehäuse (12), das die Kammer (11) enthält und eine Öffnung umfasst, durch die sich das Verbrauchsmaterial (2) aus der Kammer (11) entfernen lässt; und
einen Deckel (13), der konfiguriert ist, sich zwischen einer offenen Position, wo die Öffnung des Gehäuses (12) freigelegt ist, und einer geschlossenen Position zu bewegen, wo die Öffnung des Gehäuses (12) geschlossen ist;
wobei die Aerosolerzeugungsvorrichtung (1) geeignet ist, aus dem Verbrauchsmaterial (2) einen inhalierbaren Dampf zu erzeugen,
**dadurch gekennzeichnet, dass** der Deckel (13) ein oder mehrere Ablöseelemente (15) umfasst, die so zum Entfernen des Verbrauchsmaterials (2) geeignet sind, dass, wenn sich der Deckel (13) aus der geschlossenen Position in die offene Position bewegt:
Eine Befestigung zwischen der Kammer (11) und dem Verbrauchsmaterial (2) geschwächt wird,
und
das Verbrauchsmaterial (2) teilweise aus der Kammer (11) ausgeworfen wird, ohne am Deckel (13) zu verfangen.

2. Aerosolerzeugungsvorrichtung (1) nach Anspruch 1, wobei die Befestigung umfasst:
Reibung zwischen dem Verbrauchsmaterial (2) und der Kammer (11), und/oder
eine Verbindung zwischen dem Verbrauchsmaterial (2) und der Kammer (11), die beim Erhitzen des Verbrauchsmaterials (2) in der Kammer (11) gebildet wird.

3. Aerosolerzeugungsvorrichtung (1) nach irgendeinem vorhergehenden Anspruch, wobei die offene Position so konfiguriert ist, dass ein neues Verbrauchsmaterial (2) durch die Öffnung eingeführt werden kann, und die geschlossene Position so konfiguriert ist, dass ein Nutzer die Aerosolerzeugungsvorrichtung (1) betreiben kann, um aus dem Verbrauchsmaterial (2) den inhalierbaren Dampf zu erzeugen und diesen zu inhalieren.

4. Aerosolerzeugungsvorrichtung (1) nach irgendeinem vorhergehenden Anspruch, wobei die Ablöseelemente (15) geeignet sind, ein Flanschteil (21) des Verbrauchsmaterials (2) in Eingriff zu bringen.

5. Aerosolerzeugungsvorrichtung (1) nach Anspruch 4, wobei die Ablöseelemente (15) Schnappverbinder sind.

6. Aerosolerzeugungsvorrichtung (1) nach Anspruch 5, wobei das eine oder mehrere Ablöseelemente (15) jeweils ein Eingriffselement (151) aufweisen, das/die konfiguriert sind, sich wesentlich senkrecht zu einer Bewegungsrichtung des Deckels (13) zu bewegen, um Schnappverbindung zu bilden.

7. Aerosolerzeugungsvorrichtung (1) nach Anspruch 6, wobei das Eingriffselement (151) ein elastisches Element ist.

8. Aerosolerzeugungsvorrichtung (1) nach irgendeinem der Ansprüche 4 bis 7, wobei der Deckel (13) eine elastische Dichtung (17) umfasst, die so angeordnet ist, dass sie, wenn sich der Deckel (13) in der geschlossenen Position befindet, die elastische Dichtung (17) das Flanschteil (21) des Verbrauchsmaterials (2) kontaktiert.

9. Aerosolerzeugungsvorrichtung (1) nach Anspruch 8, wobei die Aerosolerzeugungsvorrichtung (1) einen Sensor (42) umfasst, eingerichtet zu erkennen, wenn der Deckel (13) gegen das Gehäuse (12) gepresst wird, und die elastische Dichtung (17) so angeordnet ist, dass sie den Deckel (13) vom Gehäuse (12) weg vorspannt.

10. Aerosolerzeugungsvorrichtung (1) nach irgendeinem vorhergehenden Anspruch, wobei der Deckel (13) in der offenen Position am Gehäuse (12) befestigt ist.

11. Aerosolerzeugungsvorrichtung (1) nach Anspruch 10, wobei der Deckel (13) mit einem Scharnier (16) am Gehäuse (12) befestigt ist.

12. Aerosolerzeugungsvorrichtung (1) nach Anspruch 11, wobei das Scharnier (16) eine Rastachse (161) umfasst, die so konfiguriert ist, dass der Deckel (13) in jeder der offenen Position und der geschlossenen Position stabil ist.

13. Aerosolerzeugungsvorrichtung (1) nach Anspruch 11, wobei das Scharnier (16) eine Torsionsfeder (163) umfasst, und ein Magnet (41) im Deckel (13) oder dem Gehäuse (12) enthalten ist,
wobei die Torsionsfeder (163) so konfiguriert ist, dass sie den Deckel (13) in Richtung der offenen Position vorspannt, und der Magnet (41) konfiguriert ist, den Deckel (13) in der geschlossenen Position zu halten.

14. Aerosolerzeugungsvorrichtung (1) nach irgendeinem der Ansprüche 11 bis 13, wobei ein erstes Ablöseelement (15) des einen Ablöseelements oder der mehreren Ablöseelemente so angeordnet ist, dass sich, wenn das erste Ablöseelement (15) das Verbrauchsmaterial (2) ablöst, das Verbrauchsmaterial (2) zwischen dem ersten Ablöseelement (15) und dem Scharnier (16) befindet.

15. Aerosolerzeugungsvorrichtung (1) nach irgendeinem der Ansprüche 11 bis 15, wobei die Aerosolerzeugungsvorrichtung (1) entlang einer Längsrichtung länglich ist und das Scharnier (16) senkrecht zur Längsrichtung orientiert ist.

16. Aerosolerzeugungsvorrichtung (1) nach irgendeinem vorhergehenden Anspruch, wobei das Gehäuse (12) eine Anlagefläche (121) umfasst, gegen die der Deckel (13) anliegen kann, wobei die Oberfläche die Öffnung umfasst, und die Kammer (11) im Gehäuse (12) relativ zur Anlagefläche (121) vertieft ist.

17. Aerosolerzeugungsvorrichtung (1) nach Anspruch 16, wobei das Ablöseelement (15) angeordnet ist, sich durch die Öffnung zu erstrecken, wenn sich der Deckel (13) in der geschlossenen Position befindet.

## Revendications

1. Dispositif de génération d'aérosol (1) comprenant :
une chambre (11) adaptée pour recevoir un consommable (2) ;
un boîtier (12) contenant la chambre (11) et comprenant une ouverture par laquelle le consommable (2) peut être extrait de la chambre (11) ; et
un couvercle (13) configuré pour se déplacer entre une position ouverte, dans laquelle l'ouverture du boîtier (12) est exposée, et une position fermée, dans laquelle l'ouverture du boîtier (12) est fermée ;
le dispositif de génération d'aérosol (1) étant adapté pour générer une vapeur inhalable à partir du consommable (2)
**caractérisé en ce que** le couvercle (13) comprend un ou plusieurs éléments de délogement (15) adaptés pour déloger le consommable (2) de sorte que, lorsque le couvercle (13) passe de la position fermée à la position ouverte :
une fixation est affaiblie entre la chambre (11) et le consommable (2), et
le consommable (2) est partiellement éjecté de la chambre (11) sans être alors attaché au couvercle (13).

2. Dispositif de génération d'aérosol (1) selon la revendication 1, la fixation comportant :
une friction entre le consommable (2) et la chambre (11) ; et/ou
la formation d'une liaison entre le consommable (2) et la chambre (11) lors du chauffage du consommable (2) dans la chambre (11).

3. Dispositif de génération d'aérosol (1) selon une quelconque des revendications précédentes, la position ouverte étant configurée de sorte qu'un nouveau consommable (2) puisse être inséré par l'ouverture, et la position fermée étant configurée de sorte qu'un utilisateur puisse utiliser le dispositif de génération d'aérosol (1) pour générer et inhaler la vapeur inhalable provenant du consommable (2).

4. Dispositif de génération d'aérosol (1) selon une quelconque des revendications précédentes, les éléments de délogement (15) étant adaptés pour interagir avec une partie de bride (21) du consommable (2).

5. Dispositif de génération d'aérosol (1) selon la revendication 4, les éléments de délogement (15) étant des raccords à encliquetage.

6. Dispositif de génération d'aérosol (1) selon la revendication 5, l'un ou plusieurs éléments de délogement (15) comprenant chacun un élément d'engagement (151) configuré pour se déplacer de façon substantiellement perpendiculaire à un sens du déplacement du couvercle (13), afin de constituer un raccord à encliquetage.

7. Dispositif de génération d'aérosol (1) selon la revendication 6, l'élément d'engagement (151) étant un élément élastique.

8. Dispositif de génération d'aérosol (1) selon une quelconque des revendications 4 à 7, le couvercle (13) comprenant une garniture d'étanchéité élastique (17) agencée de sorte que lorsque le couvercle (13) se trouve dans sa position fermée, la garniture d'étanchéité élastique (17) entre en contact avec la partie de bride (21) du consommable (2).

9. Dispositif de génération d'aérosol (1) selon la revendication 8, le dispositif de génération d'aérosol (1) comprenant un capteur (42) agencé pour détecter lorsque l'on appuie le couvercle (13) contre le boîtier (12), et la garniture d'étanchéité élastique (17) étant agencée pour solliciter le couvercle (13) dans le sens opposé au boîtier (12).

10. Dispositif de génération d'aérosol (1) selon une quelconque des revendications précédentes, le couvercle (13) étant fixé au boîtier (12) dans la position ouverte.

11. Dispositif de génération d'aérosol (1) selon la revendication 10, le couvercle (13) étant fixé au boîtier (12) par une charnière (16).

12. Dispositif de génération d'aérosol (1) selon la revendication 11, la charnière (16) comprenant un axe de détente (161) configuré de sorte que le couvercle (13) soit stable à chacune des positions ouverte et fermée.

13. Dispositif de génération d'aérosol (1) selon la revendication 11, la charnière (16) comprenant un ressort de torsion (163), et un aimant (41) étant incorporé dans le couvercle (13) ou le boîtier (12),
le ressort de torsion (163) étant configuré pour solliciter le couvercle (13) vers la position ouverte, et l'aimant (41) étant configuré pour maintenir le couvercle (13) en position fermée.

14. Dispositif de génération d'aérosol (1) selon une quelconque des revendications 11 à 13, un premier élément de délogement (15) de l'un ou plusieurs éléments de délogement étant agencé de sorte que, lorsque le premier élément de délogement (15) déloge le consommable (2), le consommable (2) se trouve entre le premier élément de délogement (15) et la charnière (16).

15. Dispositif de génération d'aérosol (1) selon une quelconque des revendications 11 à 15, le dispositif de génération d'aérosol (1) étant allongé dans une direction longitudinale, et la charnière (16) étant orientée perpendiculairement à la direction longitudinale.

16. Dispositif de génération d'aérosol (1) selon une quelconque des revendications précédentes, le boîtier (12) comprenant une surface de butée (121) contre laquelle le couvercle (13) peut venir buter, la surface comprenant l'ouverture, et la chambre (11) étant évidée au sein du boîtier (12) relativement à la surface de butée (121).

17. Dispositif de génération d'aérosol (1) selon la revendication 16, l'élément de délogement (15) étant agencé de façon à se déployer à travers l'ouverture lorsque le couvercle (13) se trouve dans la position fermée.
